# EUROPEAN PATENT APPLICATION

(11) **EP 2 979 726 A1**
(43) Date of publication of application: **03.02.2016**
(21) Application number: 14776454.2
(22) Date of filing: 27.03.2014
(51) Int. Cl.: A61N 1/04, C08J 9/42

(54) **POROUS SUBSTRATE ELECTRODE BODY AND METHOD FOR PRODUCING SAME**

(30) Priority: 28.03.2013 JP 2013070450
(71) Applicant: Tohoku University, Sendai-shi, Miyagi 980-8577 (JP)
(72) Inventor: NISHIZAWA, Matsuhiko, Sendai-shi Miyagi 980-8577 (JP)
(74) Representative: Gille Hrabal
(86) International application number: PCT/JP2014/058947
(87) International publication number: WO 2014/157550

(57) **Abstract**

The object of the present invention is to provide an electrode member with the hydrogel substrate, capable of producing a high-voltage pulse. Further, the other object of the present invention is to provide an electrode member which is not broken due to a deformation of the hydrogel.
The object can be solved by a porous substrate electrode member characterized in that an electrode is bound to a porous body by an adhesion layer of an electrically conducting polymer, and the electrode is at least one selected from a group consisting of a metallic electrode, a stretch electrode, a carbon electrode, and a composite electrode thereof.

## Description

### TECHNICAL FIELD

The present invention relates to a porous substrate electrode member and a method for manufacturing the same. The porous substrate electrode member can produce a high-voltage pulse, and has a high biological compatibility and flexibility.

### BACKGROUND ART

In a therapeutic instrument using electrical stimuli or a diagnostic device using electrical stimuli, an electrode coming into direct contact with skin is conventionally used. Recently, an intracorporeal embedded type medical device, a neural interface for transmitting a signal to a nerve, and the like has been developed, and further, an electrode for stimulating neurons for using therein has been developed. Furthermore, electroporation devices which introduce genes into body tissues (cells) are also developed. An electrode used in the above device may frequently come into direct contact with the body tissues or cells, and thus a material of the electrode has preferably biological compatibility.

The inventors of the present invention developed an electrode using a hydrogel as a substrate, which has biological compatibility. The hydrogel has flexibility and many of the hydrogels have high water content and a high molecular diffusivity. Thus, it has an excellent biological compatibility against cells, tissues, or the like. However, it was impossible to directly form a conductive electrode on the porous material having high water content such as a hydrogel. The inventors developed an electrode wherein an electrode pattern of an electrically conducting polymer is formed on a hydrogel substrate by electropolymerization (Patent literature 1, Non-patent literature 1 and 2).

### CITATION LIST

### PATENT LITERATURE

[Patent literature 1] WO2011/118800

### NON-PATENT LITERATURE

[Non-patent literature 1] Journal of the American Chemical Society(U.S.A.) 2010, vol.132, p.13174-13175
[Non-patent literature 2] ACS Macro Letters(U.S.A.)2012, vol.1, p.400-403

### SUMMARY OF INVENTION

### TECHNICAL PROBLEM

The present inventors have conducted intensive studies into the electrode using a hydrogel as a substrate disclosed in Patent literature 1, and Non-patent literature 1 and 2. However, the above electrode has high resistance and cannot produce a high-voltage pulse, because the electrode uses an electrically conducting polymer as a wiring material for the electrode. Further, the electrically conducting polymer per se does not have a stretch property, and therefore the electrode is sometimes broken due to a deformation of the hydrogel.

Accordingly, the object of the present invention is to provide an electrode member with the hydrogel substrate, capable of producing a high-voltage pulse. Further, the other object of the present invention is to provide an electrode member which is not broken due to a deformation of the hydrogel.

### SOLUTION TO PROBLEM

The inventors of the present invention have conducted intensive studies into the electrode using a hydrogel as a substrate, capable of producing a high-voltage pulse. As a result, the inventors, surprisingly, found that an electrode member capable of producing a high-voltage pulse can be manufactured by binding a metallic electrode or a carbon electrode to a porous body through a polymerization of an electrically conducting polymer. Further, the inventors found that a stretchable electrode member wherein the electrode is not broken, can be manufactured by binding a stretch electrode to a porous body through a polymerization of an electrically conducting polymer. The present invention is based on the above findings.

Therefore, the present invention relates to:
[1] a porous substrate electrode member wherein an electrode is bound to a porous body by an adhesion layer of electrically conducting polymer,
[2] the porous substrate electrode member characterized in that the electrode is at least one selected from a group consisting of a metallic electrode, a stretch electrode, a carbon electrode, and a composite electrode thereof,
[3] the porous substrate electrode member of the item [1] or [2], wherein the electrode is a gold, a graphite, or a conductive urethane,
[4] the porous substrate electrode member of any one of the items [1] to [3], wherein the electrode is bound to the porous body by being placed thereon or being buried therein,
[5] the porous substrate electrode member of any one of the items [1] to [4], further having an electrically conducting polymer-polymerized layer on a surface of the electrode,
[6] the porous substrate electrode member of any one of the items [1] to [5], wherein the electrically conducting polymer is at least one selected from a group consisting of poly(3,4-ethylenedioxythiophene), polyacetylene, polypyrrole, polythiophene, polybithiophene, polyisothiophene, poly dodecylthiophene, polyisonaphthothiophene, ploy(3-hexylthiophene), polyanion, polyisothianaphthene, polythiazyl, polyphenylene, polyfluorene, polydiacetylene, polyacene, polyparaphenylene, polythienylene vinylene , and polyphenylenesulfide,
[7] the porous substrate electrode member of any one of the items [1] to [6], wherein the porous body is a gel,
[8] the porous substrate electrode member of any one of the items [1] to [7], wherein the porous body is a hydrogel,
[9] the porous substrate electrode member of any one of the items [1] to [8], wherein the porous body is at least one selected from a group consisting of agarose gel, collagen gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, and a composite gel of two or more thereof,
[10] the porous substrate electrode member of any one of the items [1] to [9], wherein the porous substrate electrode member is dried and/or sterilized
[11] the porous substrate electrode member of any one of the items [1] to [10], wherein the porous substrate electrode member is for electroporation,
[12] a method for manufacturing a porous substrate electrode member, comprising the steps of:
   (1) forming an electrode on a substrate for forming an electrode,
   (2) forming a porous body so as to make contact with the electrode formed on the substrate for forming an electrode,
   (3) binding the electrode and the porous body by electropolymerizing a monomer for an electrically conducting polymer in an electrolyte solution so as to form an adhesion layer of an electrically conducting polymer from the electrode into the porous body,
   (4) separating the substrate for forming an electrode from the electrode,
[13] the method for manufacturing a porous substrate electrode member of the item 12, further comprising the step of (5) forming a polymerized layer of an electrically conducting polymer on a surface of the electrode by bringing an electrolyte solution containing a monomer for an electrically conducting polymer into contact therewith and electropolymerizing the monomer for an electrically conducting polymer,
[14] the method for manufacturing a porous substrate electrode member of the item 12 or 13, wherein the substrate for forming an electrode is a glass plate, and the electrode is at least one selected from a group consisting of a metallic electrode, a stretch electrode, and a carbon electrode,
[15] the method for manufacturing a porous substrate electrode member of any one of the items [12] to [14], wherein the substrate for forming an electrode is a glass plate, and the electrode is gold, graphite, or a conductive urethane,
[16] the method for manufacturing a porous substrate electrode member of any one of the items [12] to [15], wherein the step of forming an electrode (1) is carried out by forming the electrode on an outside surface of a rod-shaped substrate for forming an electrode, and the step of forming a porous body (2) is carried out by forming the porous body around the rod-shaped substrate for forming an electrode wherein the electrode is formed, or inserting the rod-shaped substrate for forming an electrode wherein the electrode is formed into the porous body,
[17] the method for manufacturing a porous substrate electrode member of any one of the items [12] to [16], wherein the monomer of an electrically conducting polymer is at least one selected from a group consisting of 3,4-ethylenedioxythiophene, acetylene, pyrrole, thiophene, bithiophene, isothiophene, dodecylthiophene, isonaphthothiophene, 3-hexylthiophene, anion, isothianaphthene, thiazyl, phenylene, fluorene, diacetylene, acene, paraphenylene, thienylene vinylene , and phenylenesulfide,
[18] the method for manufacturing a porous substrate electrode member of any one of the items [12] to [17], wherein the porous body is at least one selected from a group consisting of agarose gel, collagen gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, and a composite gel of two or more thereof.

### ADVANTAGEOUS EFFECTS OF INVENTION

The porous substrate electrode member of the present invention can produce a high-voltage pulse, and has a high biological compatibility and flexibility. Therefore, the porous substrate electrode member of the present invention can be used in the electrical stimuli of neurons or muscles, requiring a high-voltage pulse. Further, it can be used in an electroporation for gene transfer to cells. Furthermore, the porous substrate electrode member can be used as an implanted electrode for measurement/control of neurons or muscles.

The porous substrate electrode member using the stretch electrode of the present invention has a stretch property, and thus the electrode is not broken even if the electrode member is used in a site wherein an electrode deformation occurs. For example, the porous substrate electrode member of the present invention is also suitable for an electrode of a diagnostic or therapeutic device, which is used by attaching it to a surface of the body.

Further, a three-dimensional culture of neuron cells or muscle cells can be carried out under the electrical stimulus by using the porous substrate electrode member of the present invention wherein the electrode is buried in the porous body. Specifically, in the porous substrate electrode member wherein the stretch electrode is buried in the porous body, the porous body and the electrode have the stretch property, and thus the break of the electrode does not occur. Therefore, the three-dimensional culture can be performed while applying a mechanical stimulus. In addition, the porous substrate electrode member has the potential for use as an implanted pressure or strain sensor.

According to the electroporation using the porous substrate electrode member of the present invention, molecules can be introduced to adherent cells without a decrease of the survival rate thereof. Specifically, an air bubble formation and change of pH around the electrode can be prevented by using the electrode having an electrically conducting polymer-polymerized layer on a surface of the electrode, and thus the survival rate of cells can be increased.

### BRIEF DESCRIPTION OF THE DRAWINGS

FIG. 1 is a schematic view showing the method for manufacturing a porous substrate electrode member (in particular, a formation of gold electrode pattern) of the present invention.
FIG. 2 is a schematic view showing a binding step (polymerization of electrically conducting polymer) in the method for manufacturing a porous substrate electrode member of the present invention.

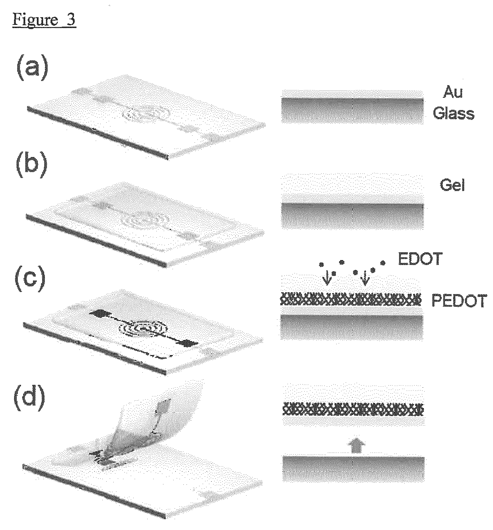

FIG. 3 is a view showing an electrode forming step (a), a porous body forming step (b), a binding step (c), and a separating step (d) in the method for manufacturing a porous substrate electrode member of the present invention.
FIG. 4 is a view showing an application of conductive urethane solution on a sacrificial layer (PVA) (a), a heat treatment (b), a binding step on the porous body (c), and a separating step (d) in the method for manufacturing a porous substrate electrode member of the present invention.
FIG. 5 is photographs showing the porous substrate electrode member comprising a polyacrylamide gel and a gold electrode obtained in Example 1 (a), and the porous substrate electrode member comprising an agarose gel and a gold electrode obtained in Example 2 (b).
FIG. 6 is a photograph showing the porous substrate electrode member comprising an agarose gel and a carbon electrode obtained in Example 3.
FIG. 7 is a photograph showing the porous substrate electrode member using urethane (stretch electrode) (A) and a graph showing the results of successive stretching which was conducted while measuring the resistance.

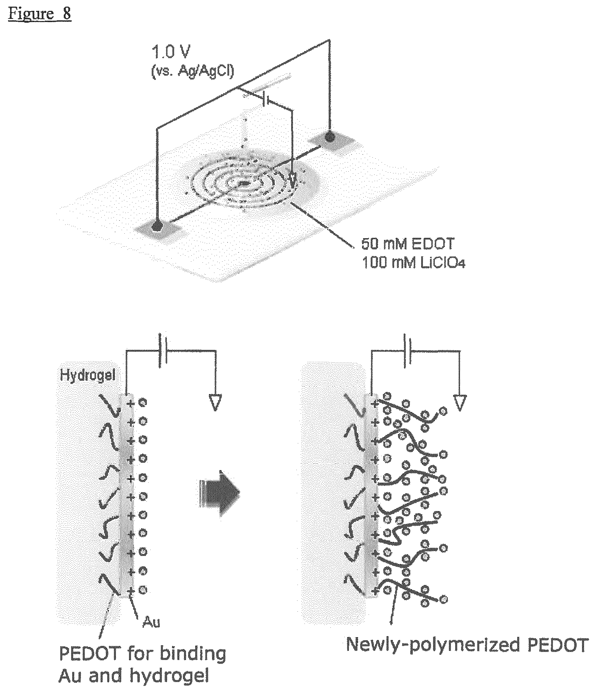

FIG. 8 is a schematic view showing the method for manufacturing the porous substrate electrode member having the electrically conducting polymer-polymerized layer.
FIG. 9 is a photograph showing the porous substrate electrode member comprising a gold electrode having an electrically conducting polymer-polymerized layer obtained in Example 5 (A) and the data showing a decrease of interface impedance using the same (B).

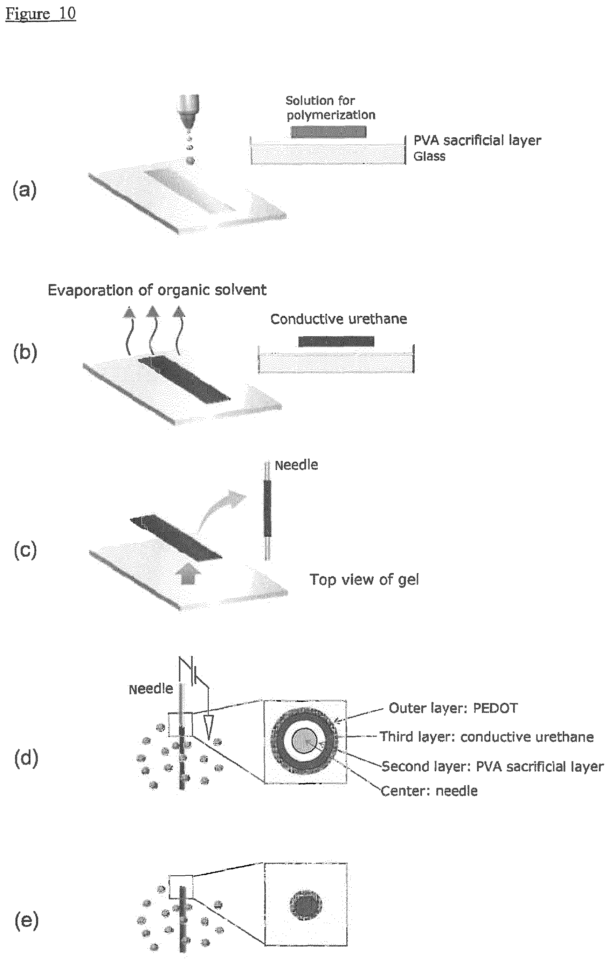

FIG. 10 is a schematic view showing the method for manufacturing a porous substrate electrode member wherein an electrode is buried in the porous body and is bound thereto by an adhesion layer of an electrically conducting polymer.
FIG. 11 is a photograph of the urethane (stretch electrode)-buried porous substrate electrode member obtained in Example 5.
FIG. 12 shows schematic views (A and B) and a photograph (C) showing an electroporation using the porous substrate electrode member and a cell-held porous body, a magnified photograph of cells between electrodes (D), a photograph showing a PI fluorescence incorporated in cells (E), and a fluorescent photograph simultaneously-observing red fluorescence derived from PI and green fluorescence derived from Calcein-AM in living cells after incorporating Calcein-AM therein (F).
FIG. 13 shows schematic views (A and B) showing an electroporation using the porous substrate electrode member with a polymerized layer and a cell-held porous body, a magnified photograph of cells between electrodes (C), a photograph showing a PI fluorescence incorporated in cells (D), and a fluorescent photograph simultaneously-observing red fluorescence derived from PI and green fluorescence derived from Calcein-AM in living cells after incorporating Calcein-AM therein (E).
FIG. 14 is photographs showing three wiring patterns (A, B, C) of the porous substrate electrode members containing stretch electrode, prepared using a spin coater and a cutting plotter.
FIG. 15 is photographs showing two wiring patterns prepared on a glass plate, using a bubble jet printer.
FIG. 16 is a photograph showing that a dried, porous substrate electrode member can be restored by being immersed in water.
FIG. 17 is photographs before (a) and after (b) sterilization by saturated water vapor under high temperature and pressure, and a graph showing electrical conductivities before or after sterilization.
FIG. 18 is photographs showing muscle cells (C2C12 cell) and locomotor neurons (NG108-15 cell) cultured on a surface of the porous substrate electrode member.
FIG. 19 is an optical photomicrograph showing that the electrode and hydrogel are strongly bound by the adhesion layer of the electrically conducting polymer.

### DESCRIPTION OF EMBODIMENTS

### [1] Porous substrate electrode member

In the porous substrate electrode member of the present invention, an electrode (preferably a metallic electrode, a stretch electrode, a carbon electrode, or a combination thereof) is bound to a porous body through an adhesion layer of an electrically conducting polymer.

Regarding a conductive body of the porous substrate electrode member, the electrode (preferably the metallic electrode, the stretch electrode, the carbon electrode, or the combination thereof) is supported by the porous body. That is, the electrode and the porous body are strongly bound by the adhesion layer of the electrically conducting polymer. A high molecular weight polymer in the adhesion layer of the electrically conducting polymer is strongly entangled with and bound to molecules of the porous body. Further, the high molecular weight polymer is electrolytically deposited on the electrode with strong binding force.

### (Electrode)

The electrode used in the porous substrate electrode member of the present invention is not particularly limited, but includes a metallic electrode, a carbon electrode, a stretch electrode, or a composite electrode thereof. As for the composite electrode, there may be mentioned a combination of metallic electrode and carbon electrode, a combination of metallic electrode and stretch electrode, or a combination of carbon electrode and stretch electrode. For example, a composite electrode wherein carbon fine particles (i.e. carbon electrode) are implanted in a surface of the stretch electrode, can be used.

### (Metallic electrode)

The metallic electrode comprised in the porous substrate electrode member of the present invention is not particularly limited, so long as it may be used as the electrode. Specifically, as for the metallic electrode, there may be mentioned gold, platinum, titanium, aluminum, or tungsten. However, gold or platinum is preferable, because these metals are stable and thus have excellent safety for the living body. Further, when the glass is used as the substrate for forming an electrode, the gold is easily separated from the glass substrate, which is preferable.

The electrical conductivity (σ(S/cm)) of the metallic electrode is not limited, but is preferably 10³∼10⁶σ(S/cm).

### (Carbon electrode)

The carbon electrode comprised in the porous substrate electrode member of the present invention is not particularly limited, so long as it may be used as the electrode. Specifically, as the carbon electrode, there may be mentioned a graphene sheet, an aggregate of carbon nanotubes, an aggregate of carbon fine particles, or a carbon fabric.

### (Stretch electrode)

The stretch electrode comprised in the porous substrate electrode member of the present invention is not particularly limited, so long as it is an elastomer which may be used as the electrode. That is to say, an electrical conductivity-applied elastomer can be used as the stretch electrode. Specifically, there may be mentioned a urethane, a silicone rubber, or a fluororubber, but the urethane is preferable. This is because the urethane has an electrical conductivity of about 10²σ(S/cm) by a combination with polythiophene.

The electrical conductivity (α(S/cm)) of the stretch electrode is not limited, but is preferably 10⁰∼10³σ(S/cm).

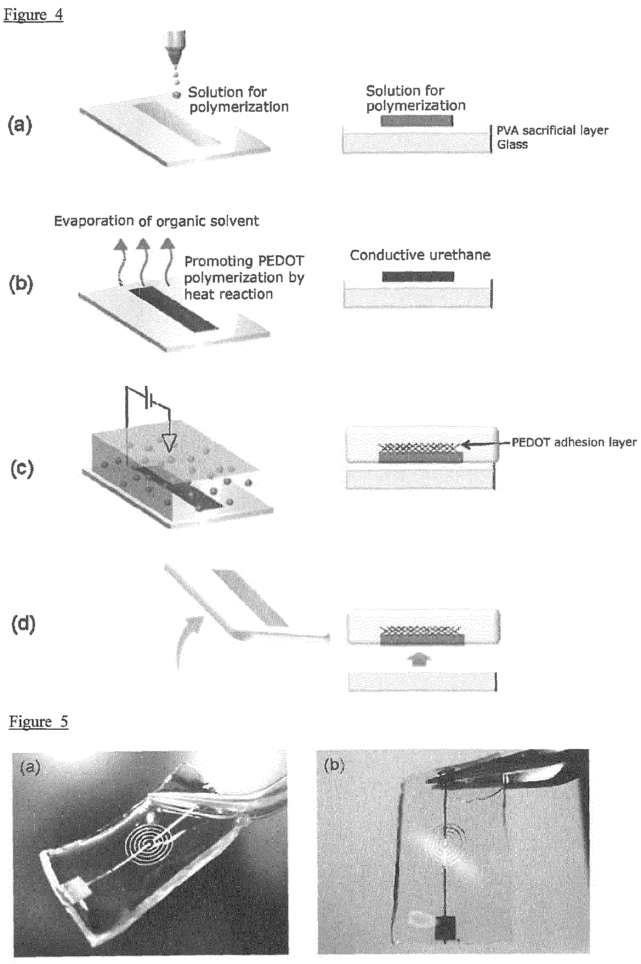

An electrode pattern of the metallic electrode and the stretch electrode is not particularly limited, it can be appropriately designed in accordance with the uses of the respective electrodes. For example, the electrode pattern illustrated in Figure 5 may be suitably used as an electrode pattern for electroporation for introducing a gene into cells.

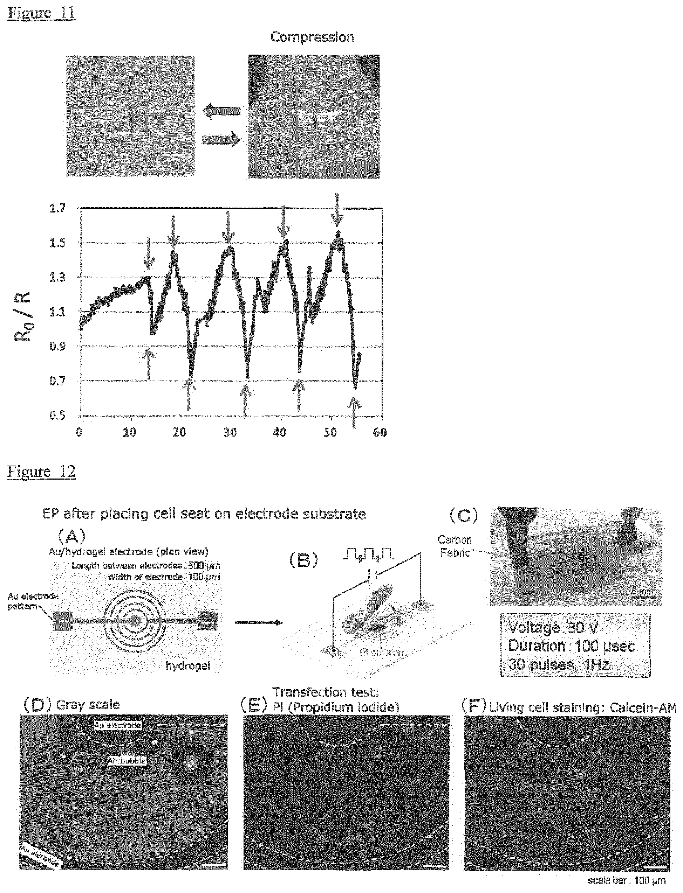

Further, the electrode illustrated in Figure 11 penetrates the cubic porous body at a hollow form. This electrode can be obtained by forming an electrode pattern on a whole surface of a rod-shaped substrate for forming an electrode, as after-mentioned.

### (Porous body)

The porous body comprised in the porous substrate electrode member of the present invention is not limited, as long as it has the stretch property, but it has, preferably, an excellent biological compatibility. For example, there may be mentioned a gel, in particular, a hydrogel is preferable.

The hydrogel contains water as a solvent in a three-dimensional network, and exhibits exceptional water absorbability. Most gels contain water regardless of whether it is natural or synthetic, and thus generally the gel means hydrogel. Further, most all of the soft tissues (such as cornea, crystalline lens, corpus vitreum, muscle, blood vessel, nerve axon, or cartilage) that constitute a living body, contain 60 to 80% of water in the network of biological macromolecule, and thus they are typical hydrogels. Furthermore, hard tissues (such as bone or tooth) are not hydrogels, per se, but most of them frequently have a structure in which a gel-like material such as collagen fills the interstices of hydroxyapatite which is an inorganic substance. Therefore, there exist many hydrogels derived from a living body or hydrogels having an excellent biological compatibility.

In particular, as for the hydrogel, there may be mentioned agarose gel, collagen gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, or a mixture (composite gel) of two or more thereof. For example, a double network gel comprising polyacrylamide and poly(2-acrylamide-2-methylpropanesulfonic acid) is strong, and thus it is largely transformable.

The porous body may contain materials other than a material that composes the porous body, as long as the effect of the present invention can be achieved. Specifically, as for the other materials, there may be mentioned cells, protein such as antibody, antigen, enzyme, or a cell growth factor, nucleic acid such as DNA or RNA, peptide molecule, micro or nano particle, fluorescent or phosphorescent molecule, redox agent, or the like. For example, the porous body containing cells, protein, peptide molecules, or nucleic acid, can be used as a medicament in a drug administration system. In the porous body containing micro or nano particle, a strength of porous body rises because the micro or nano particle acts as a cross-link point. The fluorescent or phosphorescent molecule can apply a sensing function to the porous body through a chemical response of the fluorescent or phosphorescent molecule. Further, the redox agent, the protein such as enzyme or antibody, can control the structure of the porous body by using reactivity thereof.

A water content ratio or the porous body is not particularly limited, but preferably 60 to 99.5% by weight, more preferably 70 to 99% by weight, most preferably 80 to 99% by weight.

### (Adhesion layer of electrically conducting polymer)

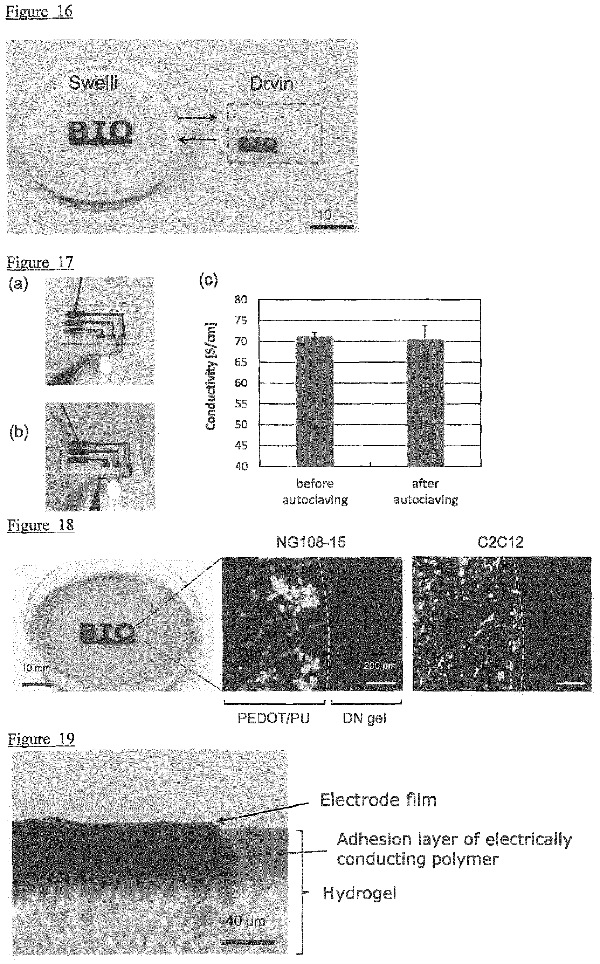

The adhesion layer of the electrically conducting polymer binds the metallic electrode, carbon electrode, or stretch electrode, and the porous body. The electrically conducting polymer elongates into an inside of the porous body from the electrode by a polymerization, whereby the electrode and the porous body are strongly bound (Figure 19).

The electrically conducting polymer comprised in the adhesion layer comprising the electrically conductingpolymer is not particularly limited, but includes poly(3,4-ethylenedioxythiophene) (hereinafter, sometimes referred to as PEDOT), polyacetylene, polypyrrole, polythiophene, polybithiophene, polyisothiophene, poly dodecylthiophene, polyisonaphthothiophene, ploy(3-hexylthiophene), polyanion, polyisothianaphthene, polythiazyl, polyphenylene, polyfluorene, polydiacetylene, polyacene, polyparaphenylene, polythienylene vinylene , Polyphenylenesulfide, or a mixture two or more thereof. However, the electrically conducting polymer is preferably Poly(3,4-ethylenedioxythiophene), polypyrrole.

For example, the PEDOT is a compound of the folowing formula (1):

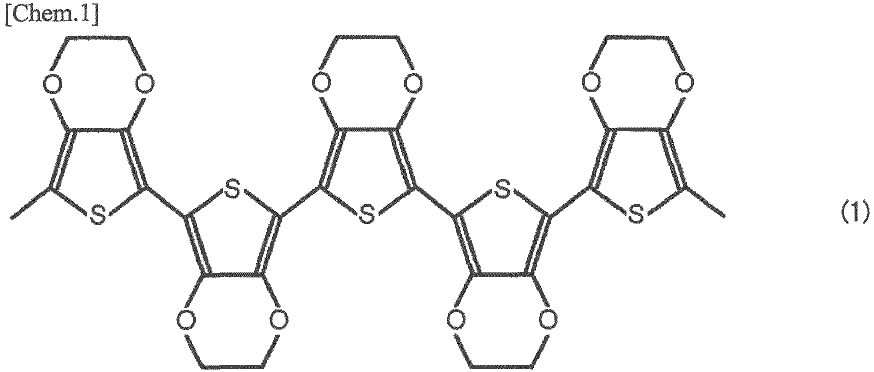

The PEDOT is an electrically conducting polymer composed of monomers wherein electron-donating alkoxy chains are introduced to the 3-position or 4 position of the thiophene. The polymerization degree of the PEDOT is not high, i.e. 5 to 10, and therefore the molecular weight thereof is approximately 1000 to 2000. However, the PEDOT exhibits a high electrical conductivity, compared to polyaniline or polypyrrole, which has a high polymerization degree.

### (Electrically conducting polymer-polymerized layer)

The porous substrate electrode member of the present invention may comprise an electrically conducting polymer-polymerized layer on the surface of the electrode. An electrode polarization hardly occurs by a presence of the electrically conducting polymer-polymerized layer. Therefore, the damage of cells or tissues due to a gas formation developed by electrolysis can be prevented.

The term "surface of electrode" as used herein means an opposite surface to the surface of the adhesion layer of electrically conducting polymer whereby the electrode binds to the porous body.

As the electrically conducting polymer used in the electrically conducting polymer-polymerized layer, the electrically conducting polymer used in the above adhesion layer of the electrically conducting polymer can be used in the same manner. Specifically, the electrically conducting polymer is not limited, but includes poly(3,4-ethylenedioxythiophene), polyacetylene, polypyrrole, polythiophene, polybithiophene, polyisothiophene, poly dodecylthiophene, polyisonaphthothiophene, ploy(3-hexylthiophene), polyanion, polyisothianaphthene, polythiazyl, polyphenylene, polyfluorene, polydiacetylene, polyacene, polyparaphenylene, polythienylene vinylene , Polyphenylenesulfide, or a mixture of two or more thereof. However, the an electrically conducting polymer is preferably poly(3,4-ethylenedioxythiophene), polypyrrole, or polyacetylene.

### (Drying)

A volume of the porous substrate electrode member of the present invention can be reduced by a drying. In addition, the reduced volume can be restored to the original volume by immersing the same in an aqueous liquid. In particular, the porous substrate electrode member using the stretch electrode has the stretch property, and thus it can be shrunk at a high rate of shrinkage, and restored.

### (Sterilization)

The porous substrate electrode member of the present invention can be sterilized and then used. A sterilization method is not particularly limited, but there may be mentioned a sterilization by saturated water vapor under high temperature and pressure (autoclave sterilization), a gaseous sterilization, a boiling water sterilization, or a sterilization by an antiseptic drug such as alcohol or hypochlorous acid. These sterilization methods can be appropriately used on a different use of the porous substrate electrode member.

As shown in the Examples, cells can be cultured on the surface of the porous substrate electrode member by using the porous substrate electrode member sterilized by the autoclave.

### [2] Method for manufacturing a porous substrate electrode member

The method for manufacturing a porous substrate electrode member of the present invention comprises the steps of: (1) forming an electrode on a substrate for forming an electrode, (2) forming a porous body so as to make contact with the electrode formed on the substrate for forming an electrode, (3) binding the electrode and the porous body by electropolymerizing a monomer for an electrically conducting polymer in an electrolyte solution so as to form an adhesion layer of an electrically conducting polymer from the electrode into the porous body, (4) separating the substrate for forming an electrode from the electrode. Preferably, the method further comprises the step of (5) forming a polymerized layer of an electrically conducting polymer on a surface of the electrode by bringing an electrolyte solution containing a monomer for an electrically conducting into contact therewith and electropolymerizing the monomer for an electrically conducting.

### (1) Electrode forming step

### (Substrate for forming electrode)

A substrate for forming an electrode is not limited, so long as a metallic electrode pattern or stretch electrode pattern can be formed thereon.

As a material of the substrate, there may be mentioned glass, plastic, cloth, or wood. However, the glass is preferable as it is smooth and has a low adhesion to the electrode.

Further, a shape of the substrate for forming an electrode is not also limited. The electrode pattern may be formed on a platy substrate, or a rod-shaped substrate.

### (Metallic electrode)

As a material of the metallic electrode, the metallic electrode material described in the above item "[1] porous substrate electrode member" can be used.

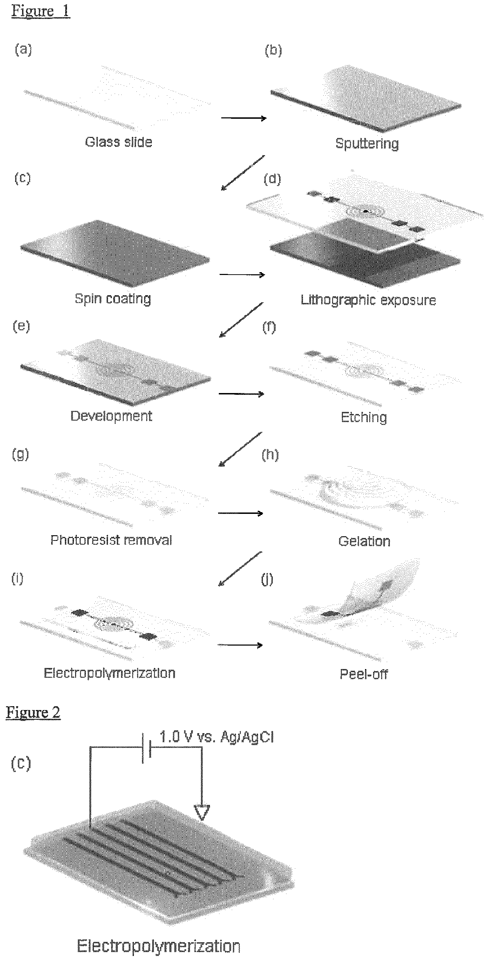

As a forming method of the metallic electrode pattern, the conventional, known methods in this technical field can be used without limitation. For example, a forming of the electrode pattern by an etching method will be illustrated according to Figure 1.
(a) A glass slide is cut into appropriate sized pieces, and they are washed for 15 minutes by ultrasonic using in order of acetone, 86% ethanol-isopropanol, and distilled water, respectively.
(b) The glass is coated with a metal such as Au by a sputter deposition.
(c) The glass substrate coated with metal is spin-coated with a positive photoresist, for example, at 4000rpm, for 30 seconds.
(d) An electrode pattern which is designed by CAD or the like, is drawn on an emulsion mask by using a laser lithography system, and it is developed to obtain a photomask.
   The photomask is placed on the glass substrate, and then it is exposed (for example, 50mJ cm⁻²) by a high-pressure mercury vapor lamp.
(e) It is developed using a developer, and washed by distilled water.
(f) The glass is immersed in an etchant such as a gold etchant, and thereby metal (Au) other than electrode pattern is removed.
(g) The photoresist is removed using acetone, and the electrode pattern is formed on a surface of the glass substrate.

In connection to this, if a metallic electrode is strongly bound to the glass slide, the metallic electrode can be easily separated from the glass slide by preliminarily applying polyvinyl alcohol on the glass slide.

### (Carbon electrode)

As a material of the carbon electrode, the carbon electrode material described in the above item "[1] porous substrate electrode member" can be used.

Methods for forming the carbon electrode are not particularly limited, but the carbon electrode can be formed as follows:
(a) A glass slide is cut into appropriate sized pieces, and they are washed for 15 minutes by ultrasonic using in the order of acetone, 86% ethanol-isopropanol, and distilled water, respectively. Then, they are stored in 2-propanol.
(b) The glass slide is spin-coated with 10 % by weight of a polyvinyl alcohol solution, for example, at 1000rpm, for 20 seconds. Then, the substrate is heated at 70°C by an oven, so as to form a polyvinyl alcohol sacrificial layer by evaporating a solvent.
(c) A dimetric frame made from a silicone sheet with a thickness of 2 mm is placed on the glass slide.
(d) A graphite solution (25mg/mL) is prepared by adding graphite powder (particle size of 3µm) to 1-Methyl-2-pyrrolidone.
(e) 200µL of the graphite solution is poured into the dimetric frame, and the solvent is evaporated at 80°C for 1 hour in an oven. Thereafter, the frame is removed so as to obtain a graphite electrode pattern substrate.

### (Stretch electrode)

As a material of the stretch electrode, the stretch electrode material described in the above item "[1] porous substrate electrode member" can be used.

Methods for forming the stretch electrode are not particularly limited. For example, a method for forming urethane can be carried out as described in Figures 4a and 4b.
(a) A glass slide is cut into appropriate sized pieces, and they are washed for 15 minutes by ultrasonic using in the order of acetone, 86% ethanol-isopropanol, and distilled water, respectively. Then, they are stored in 2-propanol.
(b) The glass slide is spin-coated with 10 % by weight of a polyvinyl alcohol solution, for example, at 1000rpm, for 20 seconds. Then, the substrate is heated at 70°C by an oven, so as to form a polyvinyl alcohol sacrificial layer by evaporating a solvent.
(c) A solution for polymerization is prepared by mixing 2mL of 1-butanol, 0.22mL of 1M EDOT monomer solution, 6.5mLof 1- butanol solution containing 400mM p-toluenesulfonic acid iron(III) (EDOT oxidant, dopant ion), 22.5mL of tetrahydrofuran solution containing 10 % by weight of polyurethane, 4.17mL of anisole (for evaporation suppression of the solvent).
(d) The solution for polymerization is applied on the glass slide in the line configuration (width of 5mm, length of 10mm) using a microinjector (EzROBO-Ace ST4040, Iwashita Engineering, Inc.), and the glass slide is heated on a hot plate at 65°C. A conductive urethane pattern is formed by accelerating an evaporation of a solvent and promoting a polymerization reaction.

### (2) Porous body forming step

In the porous body forming step, the porous body is formed so as to contact the electrode formed on the substrate for forming electrode. The porous body may be formed so that the electrode is placed on the porous body, or is buried in the porous body. Further, a preliminarily-formed porous body is arranged to contact the electrode.

As a material of the porous body, the porous body material described in the above item "[1] porous substrate electrode member" can be used.

The porous body can be formed as follows. For example, when an electrode pattern is formed on a glass slide, a frame made from a silicone sheet or the like is placed on the glass slide so as to surround the electrode pattern. A liquid containing a compound as a material of a porous body is poured into the frame, and the liquid gelates to form a porous body.

Water content ratios of gels can be determined according to each of the materials. Therefore, water content ratio is not particular limited, but preferably 60 to 99.5 % by weight, more preferably 70 to 99% by weight, most preferably 80 to 99% by weight.

The gelation of a porous body can be performed according to a publicly known method. That is to say, gelation can be performed in accordance with gelation methods of each material. Examples of gelation methods of typical materials will be described below.

### (Agarose)

Agarose is dissolved in buffer, for example, at 100°C, in the concentration of about 2.8 % by weight. The resulting sol can gelate at about 40°C by cooling the same.

### (Glucomannan)

Glucomannan gelates in an alkaline solution. For example, glucomannan powder is dissolved in water in the concentration of about 1.5 % by weight. Thereafter, 4.5 % by weight of Na₂CO₃ solution (10 % by weight) is added thereto, and mixed quickly. The mixture is poured into the frame. A glucomannan gel can be obtained by heating the mixture at 85°C for 2 hours.

### (Polyvinyl alcohol)

Polyvinyl alcohol powder is added and dissolved in heated distilled water, to obtain a 10 % by weight of a polyvinyl alcohol solution. Then, glutaraldehyde is added to the solution to give 3 % by weight of concentration, and the solution is sufficiently agitated. The solution is centrifuged at 1, 500rpm for 3 minutes so as to deform. 1M HCl solution is added thereto at a rate of 1:50, and mixed quickly. The mixture is poured into a frame, and can gelate by allowing it to stand for 12 hours.

### (Polyacrylamide)

Polyacrylamide and N,N'-ethylenebisacrylamide are added to distilled water to give 500mg/mL and 25mg/mL of concentration, respectively, and a monomer stock solution of 50 %(w/v) is prepared. The stock solution is diluted by a factor of 10, and APS and TEMED are added thereto to give 0.4 % by volume and 0.1 % by volume, respectively, and sufficiently mixed. The mixture is poured into a frame, and can gelate by allowing it to stand for 2 hours at room temperature.

### (Fibrin)

Fibrinogen is added to any solvents such as water, medium, or PBS, to obtain a solution of 30mg/mL. Thrombin is added to any solvents such as water, medium, or PBS, to obtain a solution of 20Units/mL. The above solutions are poured into a frame in equal amount, and can gelate by incubating at 37°C, for 2 to 4 hours.

### (3) Binding step

In the binding step, an adhesion layer of electrically conducting polymer is formed from the electrode to the porous body by electropolymerizing a monomer for an electrically conducting polymer to thereby bind the electrode and the porous body.

### (Monomer for electrically conducting polymer)

As a monomer for an electrically conducting polymer, the monomers which are material of the electrically conducting polymers described in the above item "[1] porous substrate electrode member" can be used. In particular, there may be mentioned 3,4-ethylenedioxythiophene (hereinafter, sometimes referred to as EDOT), acetylene, pyrrole, thiophene, bithiophene, isothiophene, dodecylthiophene, isonaphthothiophene, 3-hexylthiophene, anion, isothianaphthene, thiazyl, phenylene, fluorene, diacetylene, acene, paraphenylene, thienylene vinylene, phenylenesulfide, or a mixture thereof.

For example, the above EDOT is a compound of the following formula (2).

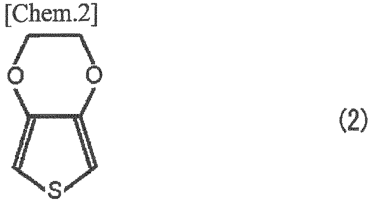

In the binding step, the monomer is dissolved in a solvent, and the solution is added to a gel of a porous body. Then, the electrically conducting polymer is electropolymerized by applying electric potential to the electrode as shown in Figure 2.

A concentration of a monomer in the solvent may be appropriately determined. For example, the concentration is 1 to 500mM, preferably 10 to 100mM.
The electric potential applied in the electropolymerization may be appropriately determined, but is preferably 0.5 to 1.5V.

A conventional oxidative electropolymerization is performed in a solution. The polymerization is started from the moment potential for polymerization is applied to a work electrode. However, in the electropolymerization of the binding step, it takes some times to diffuse the monomer for the electrically conducting polymer into hydrogel and reach a surface of work electrode, and thus a time lag between the beginning of applied potential and the beginning of polymerization is developed. For example, when an agarose gel having a thickness of 2 mm is used, the lag time from the beginning of applied potential to the beginning of polymerization is about 10 minutes. The lag time varies in accordance with the type of gel and the thickness of gel, and therefore the polymerization is preferably controlled using a coulomb meter. An amount of polymerization is not limited, so long as the electrode and the porous body may be substantially bound, but for example, preferably 100 mC/cm² to 300 mC/cm².

### (4) Separating step

In the separating step, the electrode is separated from the substrate for forming the electrode.

For example, when the Au electrode pattern is formed on the glass substrate using Au as the metallic electrode, a binding force between the Au electrode and the porous body is stronger than a binding force between the Au electrode and the glass substrate. Therefore, the porous substrate electrode member can be separated from the glass substrate by separating the porous body from the glass substrate.

Further, when a metallic electrode, carbon electrode, or stretch electrode having strong binding force to the glass substrate is used, for example, it is easily separated by preliminarily applying polyvinyl alcohol on the glass substrate, and forming the electrode pattern.

### (5) Step of forming electrically conducting polymer-polymerized layer

In the step of forming electrically conducting polymer-polymerized layer, an electrolyte solution containing a monomer for an electrically conducting polymer is brought into contact with the electrode surface, and the electrically conducting polymer-polymerized layer is formed on the electrode surface by electropolymerization.

In this step, the monomer is dissolved in a solvent, and the solution is brought into contact with the electrode surface. Then, the electrically conducting polymer is polymerized on the electrode surface by applying an electric voltage to the electrode.

A concentration of the monomer may be appropriately determined, but for example, the concentration is 1 to 500mM, preferably 10 to 100mM. An amount of solvent with respect to a used volume may be appropriately determined. The applied potential in electropolymerization may be appropriately determined, but preferably 0.5 to 1.5V. As a monomer used in this step, the electrically conducting polymer used in the above binding step can be used.

### EXAMPLES

The present invention now will be further illustrated by, but is by no means limited to, the following Examples.

### «Example 1»

In this Example, the porous substrate electrode member was prepared using the glass slide as the substrate for forming an electrode, polyacrylamide as the porous body, and gold as the electrode material.

### (1) Forming of electrode pattern (electrode forming step)

An electrode pattern was designed using Auto CAD 2990 (AutoDesk). The pattern was drawn on an emulsion mask (2 inch, FUJIFILM) by using a laser lithography system, and it was developed to obtain a photomask. A glass slide was cut into appropriately sized pieces, and they were washed for 15 minutes by ultrasonic using in the order of acetone, 86% ethanol-isopropanol, and distilled water, respectively. Then, they were stored in 2-propanol. The glass was coated with Au (300nm) by a sputter deposition (L-350-C, ANELVA). The glass substrate coated with Au was spin-coated with a positive photoresist (AZ1500, 38cp, AZ Electronic Materials) at 4000rpm for 30 seconds. The photomask was placed on the glass substrate, and then exposed at 50mJ cm⁻² by a high-pressure mercury vapor lamp. It was developed using a special developer (AZ 300MIF developer, AZ Electronic Materials), and washed twice for 30 seconds with distilled water. The glass substrate was immersed in a gold etchant (AURUM-302, KANTO Chemical Co., Inc.), and thereby Au other than electrode pattern was removed. The photoresist was removed by immersing it in acetone, and the electrode pattern was formed on a surface of the glass substrate.

### (2) Porous body forming step

A dimetric frame made from a silicone sheet with a thickness of 2 mm was placed on the glass substrate wherein the electrode pattern was formed, so as to surround the electrode pattern.

An acrylamide solution was prepared as follows. Polyacrylamide and N,N'-ethylenebisacrylamide were added to distilled water to give 500mg/mL and 25mg/mL of concentration, respectively, and thereby a monomer stock solution of 50%(w/v) was prepared. The stock solution is diluted by a factor of 10, and APS and TEMED were added thereto to give 0.4 % by volume and 0.1 % by volume, respectively, and sufficiently mixed. The mixture was poured into the frame and allowed to stand for 2 hours at room temperature. The acrylamide was polymerized to thereby form acrylamide gel.

### (3) Forming adhesion layer of electrically conducting polymer (Binding step)

EDOT monomer and LiClO₄(dopant ion) were added to distilled water to give 50mM and 100mM respectively so as to prepare a solution for polymerization. 0.2mL of the solution for polymerization was added dropwise on the porous body. An oxidative electropolymerization having 300mC/cm² of a polymerization amount was performed by applying 1.0V (vs.Ag/AgCl) of electric potential to the Au electrode and the solution for polymerization.

### (4) Separating step

After the PEDOT polymerization was completed, the acrylamide gel was separated from the substrate to obtain an acrylamide gel substrate electrode member patterned with gold.

The resulting porous substrate electrode member is shown in Figure 5(a).

### «Example 2»

In this Example, the porous substrate electrode member was prepared using the glass slide as the substrate for forming an electrode, agarose as the porous body, and gold as the electrode material.

The procedures of Example 1 were repeated except that the agarose was used instead of the acrylamide. The porous body forming step using agarose was performed as follows.

### (2) Porous body forming step

A dimetric frame made from a silicone sheet with a thickness of 2 mm was placed on the glass substrate wherein the electrode pattern was formed, so as to surround the electrode pattern.

An agarose solution was prepared as follows. Agarose powder was added to distilled water to give 2.8 % by weight, and the agarose was dissolved by heating at 100°C, to prepare agarose sol. The agarose sol was poured into the frame. An agarose gel was formed by cooling to 40°C or less.

The resulting porous substrate electrode member is shown in Figure 5(b).

### «Example 3»

In this Example, the porous substrate electrode member was prepared using the glass slide as the substrate for forming an electrode, agarose as the porous body, and graphite as the electrode material.

### (1) Forming of electrode pattern (electrode forming step)

A glass slide was cut into appropriately sized pieces, and they were washed for 15 minutes by ultrasonic using in the order of acetone, 86% ethanol-isopropanol, and distilled water, respectively. Then, they were stored in 2-propanol. The glass slide was spin-coated with 10 % by weight of a polyvinyl alcohol solution at 1000rpm for 20 seconds. Then, the substrate was heated at 70°C by an oven, so as to form a polyvinyl alcohol sacrificial layer by evaporating a solvent.

A dimetric frame made from a silicone sheet with a thickness of 2 mm was placed on the glass slide. 25mg/mL of a graphite solution was prepared by adding graphite powder (particle size of 3µm) to 1-Methyl-2-pyrrolidone. 200µL of the graphite solution was poured into the frame, and the solvent was evaporated at 80°C for 1 hour in an oven. Thereafter, the frame was removed so as to obtain a graphite electrode pattern substrate.

### (2) Porous body forming step

A dimetric frame made from a silicone sheet with a thickness of 2 mm was placed on the glass slide. An agarose solution was prepared as follows. Agarose powder was added to distilled water to give 2.8 % by weight, and the agarose was dissolved by heating at 100°C, to prepare agarose sol. The agarose sol was poured into the frame. An agarose gel was formed by cooling to 40°C or less.

### (3) Forming adhesion layer of electrically conducting polymer

EDOT monomer and LiClO₄(dopant ion) were added to distilled water to give 50mM and 100mM respectively so as to prepare a solution for polymerization. The agarose gel was cut into appropriately sized pieces, and immersed in the solution for polymerization for 30 minutes.

The agarose gel was placed on the graphite electrode pattern, and 0.2mL of the solution for polymerization was added dropwise thereon. An oxidative electropolymerization having 300mC/cm² of a polymerization amount was performed by applying 1.2V (vs.Ag/AgCl) of electric potential to the Au electrode and the solution for polymerization.

### (4) Separating step

After the PEDOT polymerization was completed, the polyvinyl alcohol sacrificial layer was dissolved by immersing the substrate in distilled water at room temperature for 5 minutes. The agarose gel was separated from the substrate to obtain an agarose gel substrate electrode member patterned with a graphite electrode.

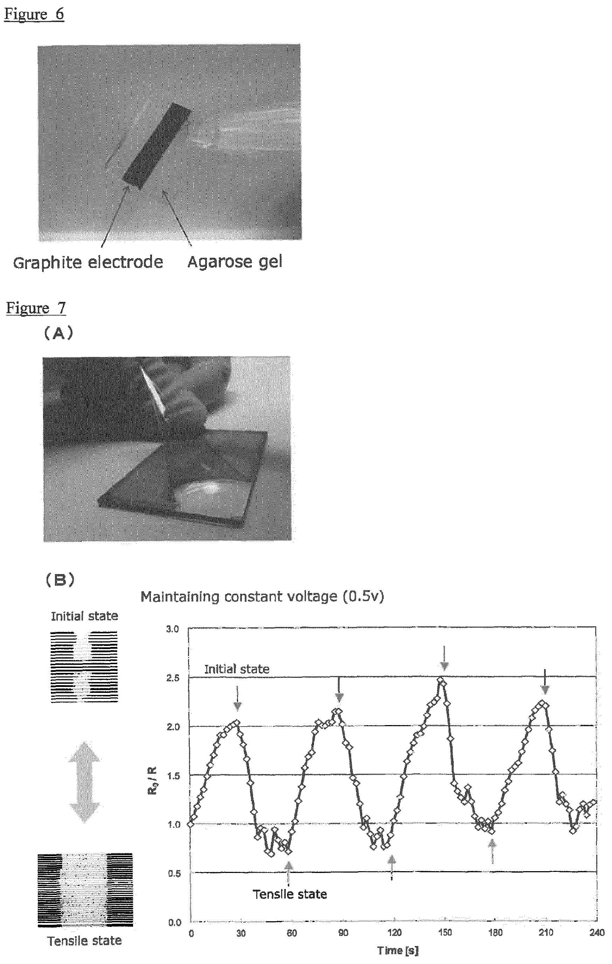

The resulting porous substrate electrode member is shown in Figure 6.

### «Example 4»

In this Example, the porous substrate electrode member was prepared using the glass slide as the substrate for forming an electrode, a double network gel comprising poly(2-acrylamide-2-methylpropanesulfonic acid) (PAMPS) gel and polyacrylamide gel as the porous body, and a conductive urethane as the electrode material.

### (1) Forming of electrode pattern (electrode forming step)

A glass slide was cut into appropriately sized pieces, and they were washed for 15 minutes by ultrasonic using in the order of acetone, 86% ethanol-isopropanol, and distilled water, respectively. Then, they were stored in 2-propanol. The glass slide was spin-coated with 10 % by weight of a polyvinyl alcohol solution at 1000rpm for 20 seconds. Then, the substrate was heated at 70°C by an oven, so as to form a polyvinyl alcohol sacrificial layer by evaporating a solvent.

A solution for polymerization was prepared by mixing 2mL of 1-butanol, 0.22mL of 1M EDOT monomer solution, 6.5mLof 1- butanol solution containing 400mM p-toluenesulfonic acid iron(III) (EDOT oxidant, dopant ion), 22.5mL of tetrahydrofuran solution containing 10 % by weight of polyurethane, and 4.17mL of anisole (for evaporation suppression of a solvent). The solution for polymerization is applied on the glass slide in the line configuration (width of 5mm, length of 10mm) using a microinjector (EzROBO-Ace ST4040, Iwashita Engineering, Inc.). The glass slide was heated on a hot plate at 65°C for 10 minutes. A conductive urethane pattern was formed by accelerating an evaporation of a solvent and promoting a polymerization reaction.

### (2) Porous body forming step

Two monomer solutions were prepared as follows. 2-acrylamide-2-methylpropanesulfonic acid, N,N'-ethylenebisacrylamide, APS, and 2-oxoglutaric acid (photopolymerization initiator) were added to distilled water to give 1M, 40mM, 0.9mM, and 2mM, respectively, and sufficiently mixed to prepare a monomer solution 1. Acrylamide, N,N'-ethylenebisacrylamide, APS, and 2-oxoglutaric acid were added to distilled water to give 1M, 1mM, 0.4mM, and 0.5mM, respectively, and sufficiently mixed to prepare a monomer solution 2.

A dimetric frame made from a silicone sheet with a thickness of 0.5 mm was placed on the glass slide. The monomer solution 1 was poured into the frame, and it gelated by being illuminated with ultraviolet (256nm of wavelength and 8W of output) at room temperature for 6 hours while being covered with a glass slide. The resulting gel was immersed into the monomer solution 2, to thereby infiltrate monomer solution 2 into the gel at 4°C for 24 hours, while blocking out light. Thereafter, the monomer solution 2 was polymerized by being illuminated with ultraviolet (256nm of wavelength and 8W of output) at room temperature for 6 hours, to obtain a double network gel.

### (3) Forming adhesion layer of electrically conducting polymer (Binding step)

The double network gel was placed on the conductive urethane pattern substrate. EDOT monomer and LiClO₄(dopant ion) were added to distilled water to give 50mM and 100mM respectively so as to prepare a solution for polymerization. The solution for polymerization was added dropwise on the porous body, and an oxidative electropolymerization having 300mC/cm² of a polymerization amount was performed by applying 1.2V (vs.Ag/AgCl) of electric potential to the conductive urethane and the solution for polymerization.

### (4) Separating step

After the PEDOT polymerization was completed, the polyvinyl alcohol sacrificial layer was dissolved by immersing the substrate in distilled water at100°C for 30 minutes. The double network gel was separated from the substrate to obtain a double network gel substrate electrode member patterned with conductive urethane.
The result of the stretch test of the resulting porous substrate electrode member is shown in Figure 7(b).

In this example, a surface of electrode was coated by an electrically conducting polymer PEDOT using the porous substrate electrode member wherein gold is used as the electrode material. The following procedures are performed after the steps in Example 1 or 2.

### (1) Step of forming electrically conducting polymer-polymerized layer

EDOT monomer and LiClO₄(dopant ion) were added to distilled water to give 50mM and 100mM respectively so as to prepare a solution for polymerization. 100µL of the solution for polymerization was added dropwise on an Au electrode surface porous body, and an oxidative electropolymerization having 300mC/cm² of polymerization amount was performed by applying 1.0V (vs.Ag/AgCl) of electric potential to the conductive urethane and the solution for polymerization to thereby form an electrically conducting polymer-polymerized layer on the surface of the electrode.

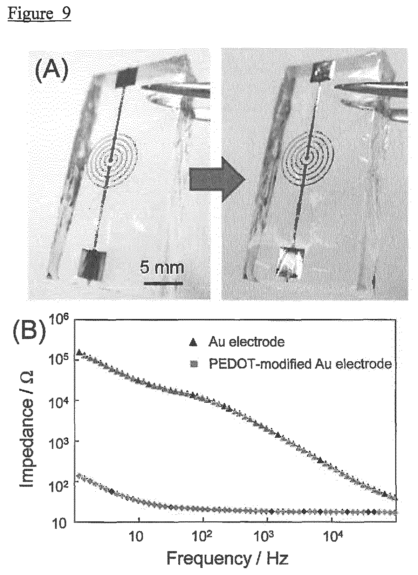

The resulting porous substrate electrode member with a polymerized layer is shown in Figure 9(a).

### (2) Impedance measurement

An AC impedance spectrum of the porous substrate electrode member was measured in 0.1M of LiClO₄ solution at the voltage amplitude of ±5mV. The result is shown in Figure 9(b).

### «Example 6»

In this example, the porous substrate electrode member was prepared using the glass slide and a needle as the substrate for forming an electrode, a double network gel as the porous body, and the conductive urethane as the electrode material.

### (1) Forming of electrode pattern (electrode forming step)

A glass slide was cut into appropriately sized pieces, and they were washed for 15 minutes by ultrasonic using in the order of acetone, 86% ethanol-isopropanol, and distilled water, respectively. Then, they were stored in isopropanol. The glass slide was spin-coated with 10 % by weight of a polyvinyl alcohol solution at 1000rpm for 20 seconds. Then, the substrate was heated at 70°C by an oven, so as to form a polyvinyl alcohol sacrificial layer by evaporating a solvent.

The solution described in Example 4(1) was used as the solution for polymerization. The solution for polymerization is applied on the glass slide in the square configuration having one square-centimeter using a microinjector (EzROBO-Ace ST4040, Iwashita Engineering, Inc.). The glass slide was heated on a hot plate at 65°C for 10 minutes. A conductive urethane was obtained by accelerating an evaporation of a solvent and promoting a polymerization reaction.

The polyvinyl alcohol sacrificial layer was dissolved by immersing the substrate in distilled water at100°C for 30 minutes so as to separate the conductive urethane from the substrate. A needle with a diameter of 1.4mm was dip-coated with 5 % by weight of a polyvinyl alcohol solution to form a sacrificial layer. Thereafter, the conductive polyurethane was wrapped around the needle to obtain a needle-shaped conductive polyurethane.

### (2) Forming adhesion layer of electrically conducting polymer (Binding step)

The double network gel described in Example 4(2) was used as the porous body. EDOT monomer and LiClO₄(dopant ion) were added to distilled water to give 50mM and 100mM respectively so as to prepare a solution for polymerization. The needle-shaped conductive urethane was immersed in the solution for polymerization while being stuck into the double network gel. An oxidative electropolymerization having 300mC/cm² of polymerization amount was performed by applying 1.2V (vs.Ag/AgCl) of the electric potential to the needle and the solution for polymerization.

### (3) Separating step

After the PEDOT polymerization was completed, the polyvinyl alcohol sacrificial layer was dissolved by immersing the porous body in distilled water at100°C for 30 minutes to separate the conductive urethane from the needle. The needle was picked from the porous body to obtain a substrate to obtain a double network gel substrate electrode member.

The result of the compression test of the resulting cubic porous substrate electrode member is shown in Figure 11.

### «Reference Example 1»

In this reference example, cells were transfected with a fluorescence reagent i.e. propidium iodide (PI) by electroporation using the porous substrate electrode member obtained in Example 1 and a cell-held porous body. PI is a cell membrane-impermeable fluorescence reagent.

### (1) Preparation of gel sheet containing cells (Step for preparing cell-held porous body)

Fibrinogen and aprotinin were added to an FBM medium to give 30mg/mL and 1mg/mL, respectively, to thereby prepare the fibrinogen solution. Thrombin was added to FBM medium to give 20Units/mL to thereby prepare thrombin solution.

Normal human dermal fibroblast cell line (NHDF-Neo) was cultured in 24 well plate to 100% confluency using a culture medium (0.5mL of hFGF-B, 10mL of FBS, 0.5mL of GA-1000(50mg/mL of gentamicin and amphotericin B of 50µg/mL) were added to 500mL of FBM medium (TAKARA BIO Inc.)). The medium was carefully aspirated from the plate in order to avoid damaging the cells. PBS(-) was gently poured thereinto using a pipet to wash cells. PBS(-) was aspirated and then equal parts of the fibrinogen solution and the thrombin solution were added to the cells. A gelation of the mixture and an adhesion of cells to the gel surface were promoted by incubating the same at 37°C for 4 hours. The fibrin gel was separated from the cell culture substrate (24 well-plates) to thereby obtain the cell-held porous body (gel sheet containing cells) wherein cells were fixed on the surface thereof.

### (2) Transfection of PI (Transfection step)

PI solution was prepared by adding PI to an electroporation buffer (Hyposmolar buffer, eppendorf) to give 1.0mg/mL. The resulting PI solution was kept in a water bath at 37°C, while blocking out light until use.

The porous substrate electrode member (hydrogel substrate electrode) obtained in Reference Example 1 was immersed in an FBM medium (TAKARA BIO Inc.) and incubated at 37°C for 2 hours. A few drops of PI solution were added dropwise on the porous substrate electrode member. The cell-held porous body (gel sheet containing cells) obtained in the above step (1) was laminated thereon so as to appress the cell surface of the cell-held porous body to an electrode surface of the porous substrate electrode member. They connected with gold electrodes of an electrode body of an electroporator (CUY21EDITII, BEX CO., LTD.), and electric pulses were applied thereon under the conditions of 80V, 0.1msec, and 30pulses (1Hz cycle).

In order to stain living cells, Calcein-AM solution was added dropwise on the gel sheet containing cells, and it was incubated in an incubator for 1 hour. The cells were fluorescently-observed using a fluorescence microscope. An evaluation of PI transfection and count of viable cells were performed. The results are shown in Figure 12.

As shown in Figure 12(E), red fluorescence derived from PI was observed in the cells that existed between electrodes. It was understood that PI was incorporated into cells. Figure 12 (F) is a fluorescence micrograph showing that red fluorescence derived from PI and green fluorescence derived from Calcein-AM were observed at the same time by transfecting Calcein-AM to living cells. Red fluorescence and green fluorescence were observed in cells located midway between electrodes. It was understood that PI was incorporated into living cells.
However, cells near the electrodes, and cells near the air bubbles shown in Figure 12(D) have weak green fluorescence, and thus the above cells were considered the dead cells. A survival rate of cells was about 50%.

### «Reference Example 2»

In this reference example, adherent cells were transfected with PI using the porous substrate electrode member obtained in Example 5.

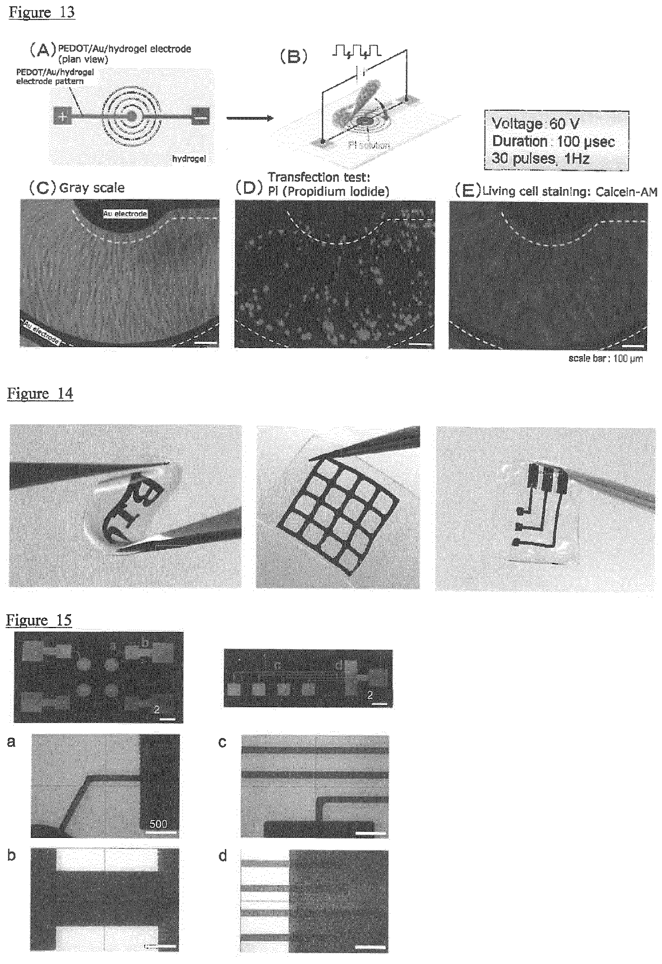

The procedures of Reference Example 1 were repeated except that the porous substrate electrode member with a polymerized layer obtained in Example 5 was used instead of the porous substrate electrode member obtained in Example 1, and the pulse conditions were 60V, 0.1msec, and 30 pulse (1Hz cycle). The results are shown in Figure 13.

Red fluorescence derived from PI were observed in the cells that existed between electrodes. That is, PI was incorporated into cells. Further, as shown in Figure 13(C), air bubbles did not develop in the area near the electrodes. Figure 13(E) is a fluorescence micrograph showing that red fluorescence derived from PI and green fluorescence derived from Calcein-AM were observed at the same time by transfecting Calcein-AM to living cells. Most of PI-transfected cells were living cells. A survival rate of cells was more than 80%. That is to say, the survival rate of cells can be increased using the porous substrate electrode member with a polymerized layer.

### «Example 7»

In this Example, the porous substrate electrode member was prepared using the glass slide as the substrate for forming an electrode, a double network gel comprising poly(2-acrylamide-2-methylpropanesulfonic acid) (PAMPS) gel and polyacrylamide gel as the porous body, and a conductive urethane as the electrode material. An electrode pattern was prepared.

An electrode pattern was prepared using a spin coater and a cutting plotter.

### (1) Forming of electrode pattern (electrode forming step)

A glass slide was cut into appropriately sized pieces, and they were washed for 15 minutes by ultrasonic using in the order of acetone, 86% ethanol-isopropanol, and distilled water, respectively. Then, they were stored in isopropanol. The glass slide was spin-coated with 10 % by weight of a polyvinyl alcohol solution at 1000rpm for 20 seconds. Then, the substrate was heated at 70°C by an oven, so as to form a polyvinyl alcohol sacrificial layer by evaporating a solvent.

A solution for polymerization (EDOT/PU solution) was prepared by mixing 2mL of 1-butanol, 0.22mL of 1M EDOT monomer solution, 6.5mLof 1- butanol solution containing 400mM p-toluenesulfonic acid iron(III) (EDOT oxidant, dopant ion), 22.5mL of tetrahydrofuran solution containing 10 % by weight of polyurethane, and 4.17mL of anisole (for evaporation suppression of a solvent). The solution for polymerization is applied on the glass slide using the spin coater at 750rpm for 30minutes. The glass slide was heated on a hot plate at 65°C for 10 minutes. A conductive urethane pattern was formed by accelerating an evaporation of a solvent and promoting a polymerization reaction. The glass slide was heated on a hot plate at 100°C for 10 minutes. A thin film of conductive urethane (PEDOT/PU) was formed by accelerating an evaporation of a solvent and promoting a polymerization reaction.

The thin film was cut by the cutting plotter to obtain a desired pattern. Unnecessary parts of PEDOT/PU thin film were removed in water by using a pincette to obtain a wiring pattern of PEDOT/PU.

The procedures of (2) Porous body forming step, (3) Forming adhesion layer of electrically conducting polymer (Binding step), and (4) Separating step described in Example 4 were repeated to obtain a porous substrate electrode member (Figure 14).

### «Example 8»

In this Example, the electrode pattern was prepared using the glass slide as the substrate for forming an electrode and an ink jet printer (Figure 15).

### (1) Forming of electrode pattern (electrode forming step)

A glass slide was cut into appropriately sized pieces, and they were washed for 15 minutes by ultrasonic using in the order of acetone, 86% ethanol-isopropanol, and distilled water, respectively. Then, they were stored in isopropanol. The glass slide was spin-coated with 10 % by weight of a polyvinyl alcohol solution at 1000rpm for 20 seconds. Then, the substrate was heated at 70°C by an oven, so as to form a polyvinyl alcohol sacrificial layer by evaporating a solvent.
A solution for polymerization (EDOT/PU solution) was prepared by mixing 2mL of 1-butanol, 0.22mL of 1M EDOT monomer solution, 6.5mL of 1- butanol solution containing 400mM p-toluenesulfonic acid iron(III) (EDOT oxidant, dopant ion), 22.5mL of tetrahydrofuran solution containing 10 % by weight of polyurethane, and 4.17mL of anisole (for evaporation suppression of a solvent). An ink jet printer was filled with the EDOT/PU solution. A wiring pattern was drawn on the glass slide on a hot plate at 100°C to obtain a wiring pattern of PEDOT/PU.

### «Example 9»

In this Example, the porous substrate electrode member obtained in Example 7 was dried.

The porous substrate electrode member was dried by being allowed to stand at 25°C, in air, 12 hours, as a result, a volume of the porous substrate electrode member was reduced to a quarter. However, the reduced volume was recovered to the original volume by immersing the dried porous substrate electrode member in water (Figure 16). A detachment of the electrode in the porous substrate electrode member was not observed. Further, when the current was applied to the porous substrate electrode member, an elevation of resistance was not observed.

### «Example 10»

In this example, the porous substrate electrode member obtained in Example 7 was sterilized.

Specifically, the porous substrate electrode member was heated for 20 minutes under high temperature and pressure (121°C, 2 atmospheres), saturated water vapor. However, the detachment of the electrode in the porous substrate electrode member was not observed. Further, in the porous substrate electrode members before (Figure 17a) and after (Figure 17a) sterilization, a change of electrical conductivity was not observed (Figure 17c).

### <<Example 11>>

In this example, cells were cultured on the porous substrate electrode member sterilized in Example 10.

C2C12 cells (muscle cells) or NG108-15 cells (locomotor neurons) were cultured on the surface of the porous substrate electrode member, to thereby evaluate a cellular adhesiveness to the electrode surface. The results are shown in Figure 18. Even though a cell adhesion protein or an adherence factor was not used, both cells adhere to the surface of the porous substrate electrode member and proliferate almost without cell death. Furthermore, even when the culture conditions were maintained for a few days, an increase of cell death cannot be observed. Therefore, the porous substrate electrode member of the present invention has an excellent biological compatibility (non-toxicity) to cells. As shown with an arrow in Figure, in the case of the neurons, it was observed that the cell elongates long neurites in a direction toward a neighbor cell, and thus, it was understood that the porous substrate electrode member can be used as the substrate highly-effective for the cell-to-cell interaction.

### «Photographing of adhesion layer of electrically conducting polymer»

A photograph of an interface between the electrode and the hydrogel of the porous substrate electrode member obtained in Example 7 was taken by a light microscope. As shown in Figure 19, it was understood that the electrode and the hydrogel were strongly bound by the adhesion layer of electrically conducting polymer.

### INDUSTRIAL APPLICABILITY

The porous substrate electrode member of the present invention can be used as an electrode pattern for electroporation, an electrode of a diagnostic or therapeutic device, which is used by attaching it to a surface of the body, an implanted electrode for measurement/control of neurons or muscles, an electrode for culturing neurons or muscle cells, or an electrode for implanted pressure or strain sensor.

Although the present invention has been described with reference to specific embodiments, various changes and modifications obvious to those skilled in the art are possible without departing from the scope of the appended claims.

## Claims

1. A porous substrate electrode member wherein an electrode is bound to a porous body by an adhesion layer of electrically conducting polymer.

2. The porous substrate electrode member **characterized in that** the electrode is at least one selected from a group consisting of a metallic electrode, a stretch electrode, a carbon electrode, and a composite electrode thereof.

3. The porous substrate electrode member according to claim 1 or 2, wherein the electrode is a gold, a graphite, or a conductive urethane.

4. The porous substrate electrode member according to any one of claims 1 to 3, wherein the electrode is bound to the porous body by being placed thereon or being buried therein.

5. The porous substrate electrode member according to any one of claims 1 to 4, further having an electrically conducting polymer-polymerized layer on a surface of the electrode.

6. The porous substrate electrode member according to any one of claims 1 to 5, wherein the electrically conducting polymer is at least one selected from a group consisting of poly(3,4-ethylenedioxythiophene), polyacetylene, polypyrrole, polythiophene, polybithiophene, polyisothiophene, poly dodecylthiophene, polyisonaphthothiophene, ploy(3-hexylthiophene), polyanion, polyisothianaphthene, polythiazyl, polyphenylene, polyfluorene, polydiacetylene, polyacene, polyparaphenylene, polythienylene vinylene , and polyphenylenesulfide.

7. The porous substrate electrode member according to any one of claims 1 to 6, wherein the porous body is a gel.

8. The porous substrate electrode member according to any one of claims 1 to 7, wherein the porous body is a hydrogel.

9. The porous substrate electrode member according to any one of claims 1 to 8, wherein the porous body is at least one selected from a group consisting of agarose gel, collagen gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, and a composite gel of two or more thereof.

10. The porous substrate electrode member according to any one of claims 1 to 9, wherein the porous substrate electrode member is dried and/or sterilized.

11. The porous substrate electrode member according to any one of claims 1 to 10, wherein the porous substrate electrode member is for electroporation.

12. A method for manufacturing a porous substrate electrode member, comprising the steps of:
(1) forming an electrode on a substrate for forming an electrode,
(2) forming a porous body so as to make contact with the electrode formed on the substrate for forming an electrode,
(3) binding the electrode and the porous body by electropolymerizing a monomer for an electrically conducting polymer in an electrolyte solution so as to form an adhesion layer of an electrically conducting polymer from the electrode into the porous body,
(4) separating the substrate for forming an electrode from the electrode.

13. The method for manufacturing a porous substrate electrode member according to claim 12, further comprising the step of (5) forming a polymerized layer of an electrically conducting polymer on a surface of the electrode by bringing an electrolyte solution containing a monomer for an electrically conducting polymer into contact therewith and electropolymerizing the monomer for an electrically conducting polymer.

14. The method for manufacturing a porous substrate electrode member according to claim 12 or 13, wherein the substrate for forming an electrode is a glass plate, and the electrode is at least one selected from a group consisting of a metallic electrode, a stretch electrode, and a carbon electrode.

15. The method for manufacturing a porous substrate electrode member according to any one of claims 12 to 14, wherein the substrate for forming an electrode is a glass plate, and the electrode is gold, graphite, or a conductive urethane.

16. The method for manufacturing a porous substrate electrode member according to any one of claims 12 to 15, wherein the step of forming an electrode (1) is carried out by forming the electrode on an outside surface of a rod-shaped substrate for forming an electrode, and the step of forming a porous body (2) is carried out by forming the porous body around the rod-shaped substrate for forming an electrode wherein the electrode is formed, or inserting the rod-shaped substrate for forming an electrode wherein the electrode is formed into the porous body.

17. The method for manufacturing a porous substrate electrode member according to any one of claims 12 to 16, wherein the monomer of an electrically conducting polymer is at least one selected from a group consisting of 3,4-ethylenedioxythiophene, acetylene, pyrrole, thiophene, bithiophene, isothiophene, dodecylthiophene, isonaphthothiophene, 3-hexylthiophene, anion, isothianaphthene, thiazyl, phenylene, fluorene, diacetylene, acene, paraphenylene, thienylene vinylene, and phenylenesulfide.

18. The method for manufacturing a porous substrate electrode member according to any one of claims 12 to 17, wherein the porous body is at least one selected from a group consisting of agarose gel, collagen gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, and a composite gel of two or more thereof.
